# EUROPEAN PATENT APPLICATION

(11) **EP 4 354 458 A1**
(43) Date of publication of application: **17.04.2024**
(21) Application number: 22203325.0
(22) Date of filing: 24.10.2022
(51) Int. Cl.: G16H 50/70, G16H 70/00, G16H 10/60, G16H 40/20

(54) **GENERATING CLINICAL PATHWAY ANALYTICS**

(30) Priority: 14.10.2022 US 202263416282 P
(71) Applicant: Koninklijke Philips N.V., 5656 AG Eindhoven (NL)
(72) Inventor: NALLURI, Joseph Jayakar, Eindhoven (NL); VAN OMMERING, Rob, Eindhoven (NL); SCHUURKAMP, Gertjan Laurens, 5656AG Eindhoven (NL)
(74) Representative: Philips Intellectual Property & Standards

(57) **Abstract**

A method is provided for generating and displaying clinical pathway analytics. The method comprises obtaining clinical data of a pathology for a plurality of patients and identifying treatment-oriented steps from the clinical data forming a plurality of pathways. Correlated metrics are then determined between treatment-oriented steps in each pathway from the clinical data. The clinical pathway analytics are generated by using the treatment-oriented steps as nodes and generating links between the treatment-oriented steps in each pathway, where one or more visual parameters of the links correspond to the correlated metrics.

## Description

### FIELD OF THE INVENTION

The invention relates to the field of clinical pathways and graphical analytics viewers for care pathways. In particular, the invention relates to generating and displaying analytics for said pathways.

### BACKGROUND OF THE INVENTION

A clinical pathway is a quality-driven treatment protocol or workflow designed to reduce unwarranted variation in care and thereby reduce costs and/or improve clinical outcome and operational efficiency. Clinical oncology pathways are evidence-based specific treatment plans driven by research studies and best practices.

Clinical pathways can cater to a patient diagnosed with, for example, a cancer, which might include parameters such as cancer type, stage and prognosis. Pathways are designed to deliver standardized high quality treatment for patients and reduce the treatment variability while lowering overall healthcare costs. With the shift in the healthcare industry to deliver value-based care, oncology pathways have garnered heavy acceptance across several healthcare institutions. Several insurance companies and hospitals have adopted the usage of clinical cancer pathways or cardiology care pathways. When using such pathways, every patient undergoes a navigation of treatment plan based on his/her condition via pathways. Hence, in order to observe the performance of pathways-based treatment management across a population of patients, analytics is required.

Graphical analytics viewers of the clinical pathways are the most intuitive way for a physician to comprehend the available treatment plans and corresponding choices to make a clinically informed decision.

The care provided by the pathways is dispersed, which means that the adoption of such across healthcare institutions varies. In order to qualify as a high-quality pathways program and thus increasing the clinical acceptance, the American Society of Clinical Oncology (ASCO) has certain standards. One of the standards and criteria is the ability of the platform vendor to conduct analytics, that the data is comprehensive yet transparent, and has clear and achievable expected outcomes. However, since the data is dispersed and has multiple complexities to be analyzed, it is difficult to achieve an accepted clinical pathway according to ASCO criteria.

Thus, there is a need to improve clinical pathways such that they continue being intuitive whilst further being able to conduct analytics to aid in the decision-making process of clinicians and providing input for policy changes and/or influencing better payment models between healthcare providers and payers.

### SUMMARY OF THE INVENTION

The invention is defined by the claims. Dependent claims represent beneficial embodiments of the invention.

According to examples in accordance with an aspect of the invention, there is provided a method for generating and displaying clinical pathway analytics, the method comprising:
obtaining clinical data corresponding to a pathology or disease in respect of a plurality of patients;
identifying a plurality of treatment-oriented steps from the clinical data forming a plurality of pathways;
determining correlated metrics between treatment-oriented steps in the pathways from the clinical data;
generating the clinical pathway analytics by:
   representing the treatment-oriented steps as nodes; and
   generating links between the treatment-oriented steps in the pathways, wherein one or more visual parameters of the links correspond to the correlated metrics; and
displaying the clinical pathway analytics to a user.

Clinicians often have to make decisions regarding which treatment a particular patient with a particular pathology or disease will follow. Clinicians may rely on their previous experience, as well as their knowledge of the pathologies and diseases, to decide on the treatment course. However, this assumes that the clinician has enough experience on that particular pathology. Even in that case, the decision is potentially error-prone due to the complexity of the task and the overwhelming amount of information. The chance of a correct diagnosis and treatment selection is even lower with less experienced clinicians or with particularly rare pathologies. Person having ordinary skill in the art would understand, that the term "pathology" is applied in a broad sense, and this is a term may mean anatomical pathology of a patient, molecular pathology, anatomical pathology, disease of a patient, cancer type, etc.

Clinical pathways (i.e. clinical pathway analytics) often provide a lot of useful information relating to the corresponding pathology. In particular, the clinical pathways may show all possible treatment options for the pathology at hand. Often, however, different treatment options appear in similarly looking boxes without putting enough emphasis on the recommended choice for a specific case at hand. It has been realized that such visual representation of the clinical pathway makes it difficult for the clinician to properly appreciate all possibilities and make a decision based on all possible treatments and the amount of criteria to consider.

In one example, it is proposed to determine correlated metrics between the treatment-oriented steps and visually adapt the links between the nodes to correspond to the correlated metrics. The correlated metrics are values or statistics which relate to patients on a treatment pathway which includes that particular path, i.e. the correlated metrics apply to those patients on a path between a particular pair of the treatment-oriented steps. The visual adaptations provide a variation in the look of the clinical pathway. This provides information on the statistics calculated between different treatment-oriented options or steps obtained from the clinical data. The visual adaptations provide an easily accessible information source from the clinical data which can be used to make a clinical decision (e.g. how many people go through each treatment-oriented step). As the additional statistical information has been incorporated by adapting the visual parameters, the clinical pathway will still be intuitive to the clinician and would ensure that fewer clinical errors would be made by the clinician. Furthermore, since the statistical parameters would calculate the preferred treatment option for a specific patient considering specific parameters at hand (treatment preferences of a specific clinical institution, previous cases, parameters relevant for a specific disease, probabilities, etc.), this would eventually ensure improved clinical care. As an additional advantage, this may improve machine operations due to e.g. more efficient clustering.

Further, the variation in the visual parameters (e.g. color, size, transparency, texture etc.) of the clinical pathway may mean that the clinician is more likely to look through all of the treatment-oriented steps as they will have a distinct look and thus be more visually interesting. This would decrease the number of clinical errors in the clinical institution.

Additionally, it is important for key leadership stakeholders at major hospitals to have the ability to analyze the efficacy and performance of particular pathways in order to understand operational bottlenecks and performance issues, as well as increasing standard of care in the clinical institution. Conventional clinical pathway analytics can be hard to comprehend as a lot of data is typically presented. Furthermore, this increases the difficulties of performing clinical statistics.

Thus, the correlation of the visual parameters to the correlated metrics provides an indication of the relative statistical performance of each pathway and previous treatment preferences for similar cases thereby providing an intuitive representation of the clinical pathway analytics.

Treatment-oriented steps can include clinical attributes and criteria (e.g. smoker, weight, previous diseases, present gene mutations, etc.), actions to be taken, and conditions for putting a patient on a pathway ("on-pathway)". These are typically used by a physician to proceed towards the treatment. In one embodiment, the treatment-oriented steps can include at least one of: the disease type, receptor indications (positive, negative for a specific cancer type), localization of the cancer.

The correlated metrics may include the number of said patients who pass between the associated treatment-oriented steps. The correlated metrics may include the number of said patients subject to the associated treatment-oriented steps.

In general, the correlated metrics include statistical data associated to the treatment-oriented steps.

The one or more visual parameters may comprise one or more of: a line width of the links, color of the links, dashed lines and doubled lines. The links could refer to a statistical probability of a specific pathway.

A linear or log scale could be used, for example, for the line width in respect of the number of patients who pass between the associated treatment-oriented steps.

The method may further comprise determining pathway metrics for one or more of the treatment-oriented steps and displaying the pathway metrics to the user.

The pathway metrics may comprise one or more of: whether the treatment-oriented step is on-pathway or off-pathway, why the treatment-oriented step is off-pathway, cost of the treatment-oriented step, survival rate of the treatment-oriented step, clinical outcomes of the treatment-oriented step, treatment patterns, treatment usage data, population characteristics, treatment outcomes, probability score, outcomes from treatment-oriented step and cohort identification.

As will be apparent for the person having ordinary skill in the art, further visual parameters, correlated metrics, or treatment-oriented steps can be used within the context of the present invention.

In an example for treatment patterns, there may be several "on-pathway" treatment regimens organized in a top-down list. Identifying which among the "on-pathway" treatment courses the physicians select most often can give insight into physician's preferences or biases. This might assist leadership to identify and address that appropriately.

In an example for treatment usage data, when observing aggregated data, the utilization rate of a particular treatment course (consisting of drug regimens) can be used to forecast drug utilization for the pharmacy. The pharmacy can use that forecast to pre-order or refill their stock of medications.

In an example for population characteristics, the age, gender, ethnicity, race and other characteristics can be represented. In an example, consider the case of a clinical pathway for a patient with cancer. Cancer is a disease of the genomic alterations and mutations of the cells. Population groups are diverse in their attributes such as age, gender, ethnicity, race, etc. Being able to show how different populations have undergone and reacted to different therapies gives insight to the physicians while choosing medication for a prospective patient.

Furthermore, several metrics can be used to quantify the treatment outcome, for instance after the treatment has begun. Examples are quality of life, disease-free survival, progression-free survival, toxicity scores, adverse event, etc. Being able to show these outcomes for aggregated data (across institution/regions) to the physicians is useful for them while deciding the best course of treatment for the prospective patient

The probability score is a probability that the current patient will encounter any of the aforementioned treatment outcomes, if prescribed a certain medication. Using aggregated data (containing past patients and their outcomes), prediction algorithms can derive a probability score for a prospective patient to encounter a certain treatment outcome. Kaplan-Meier survival curves (product limit estimator) are one such example.

At each treatment-oriented step, certain metrics (such as on/off pathway rate) can be shown based on the aggregated data at a specific point in time. Instead of showing all the metrics at the end of the pathway, some metrics can be shown much earlier in the pathway tree. It essentially shows the utilization of the pathway across the displayed metrics. In one embodiment, only the most probable clinical pathway regimes and metrics can be shown to the user for easy review. In another embodiment, the user can collapse or expand the pathway tree for review of suggested statistics.

In one example of the cohort identification, consider a pathway where each treatment-oriented step is either is a condition or an action. For e.g., Lung Non-Small Cell Cancer >> Metastatic >> Non-squamous >> ALK mutation present>> No prior ALK Inhibitor administered >> Prescribe treatment XYZ. Herein, the node 'ALK mutation present' represents the number of patients meeting the condition, thereby identifying the patient cohort consisting of ' lung non-small cell cancer, disease state of metastatic, histology of non-squamous and ALK mutation'. The one of ordinary skill in the art would understand that ALK refers to mutations in relation to ALK gene.

In one example, displaying the pathway metrics may comprise overlaying the pathway metrics on top of the corresponding treatment-oriented step. This may occur when the user interacts with the treatment-oriented step on the displayed clinical pathway analytics. Other examples may be possible within the context of the present invention.

This enables the user to have as much information as required for each treatment-oriented step without the need for displaying all of the information at once. The user only needs to interact with the desired treatment-oriented step to see all of the corresponding pathway metrics.

The method may further comprise determining a recommended pathway from the plurality of pathways and displaying the treatment-oriented steps and/or links corresponding to the recommended pathway. The recommended pathway may be displayed with different visual parameters than the other treatment-oriented steps and/or links. In one example, the recommended pathway can be highlighted or otherwise emphasized.

The invention also provides a computer program product comprising computer program code which, when executed on a computing device having a processing system, causes the processing system to perform all of the steps of the method as described in the present application.

The invention also provides a system for generating and displaying clinical pathway analytics, the system comprising a processor configured to:
obtain clinical data corresponding to a pathology in respect of a plurality of patients;
identify a plurality of treatment-oriented steps from the clinical data forming a plurality of pathways;
determine correlated metrics between treatment-oriented steps in pathways from the clinical data;
generate the clinical pathway analytics by:
   representing the treatment-oriented steps as nodes; and
   generating links between the treatment-oriented steps in pathways, wherein one or more visual parameters of the links correspond to the correlated metrics; and control a display to display the clinical pathway analytics to a user.

The correlated metrics may include the number of said patients who pass between the associated treatment-oriented steps.

The one or more visual parameters may comprise one or more of: a line width of the links, color of the links, dashed lines and doubled lines.

The processor may be further configured to determine pathway metrics for one or more of the treatment-oriented steps and displaying the pathway metrics to the user.

The pathway metrics may comprise one or more of: whether the treatment-oriented step is on-pathway or off-pathway, why the treatment-oriented step is off-pathway, cost of the treatment-oriented step, survival rate of the treatment-oriented step, clinical outcomes of the treatment-oriented step, treatment patterns, treatment usage data, population characteristics, treatment outcomes, probability score, outcomes from treatment-oriented step and cohort identification.

The processor may be configured to display the pathway metrics by overlaying the pathway metrics on top of the corresponding treatment-oriented step. This may occur when the user interacts with the treatment-oriented step on the displayed clinical pathway analytics.

The processor may be further configured to determine a recommended pathway from the pathways and display the treatment-oriented steps and/or links corresponding to the recommended pathway. The recommended pathway may be displayed with different visual parameters than the other treatment-oriented steps and/or links.

These and other aspects of the invention will be apparent from and elucidated with reference to the embodiment(s) described hereinafter.

One of the advantages of the present invention is that the treatment outcomes can be improved by reducing the error rate.

Another advantage of the present application is that the clinical workflow can be improved since the physicians would spend less time per patient.

Another advantage of the present application is improved representation of data to the clinician, and associated improvement of the data structuring.

Another advantage of the present application is the improvement of the data structuring for the clinical pathways.

The one of the ordinary skill in the art would appreciated that other advantages within the context of the invention can be envisioned.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an existing oncology cancer pathway for colorectal cancer;
Fig. 2 shows a snapshot of a bar chart showing the number of patients for each treatment-oriented step;
Fig. 3 shows an exemplary clinical pathway according to the invention; and
Fig. 4 shows a portion of a clinical pathway showing pathway metrics overlaid on the clinical pathway.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for generating and displaying clinical pathway analytics. The method comprises obtaining clinical data of a pathology for a plurality of patients and identifying treatment-oriented steps from the clinical data forming a plurality of pathways. Correlated metrics are then determined between treatment-oriented steps in each pathway from the clinical data. The clinical pathway analytics are generated by using the treatment-oriented steps as nodes and generating links between the treatment-oriented steps in each pathway, where one or more visual parameters of the links correspond to the correlated metrics.

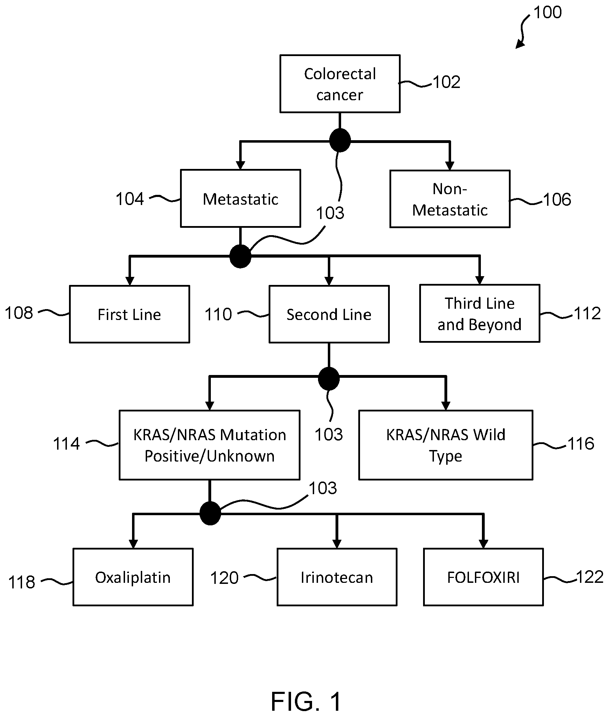

Fig. 1 shows an existing oncology cancer pathway 100 for colorectal cancer. Each box in the tree-based pathway 100 is an attribute of the patient's condition that warrants the subsequent treatment plan.

In this case, attribute 102 corresponds to "Colorectal cancer". The attributes 104 and 106, following attribute 102, are "metastatic" and "non-metastatic". Only the pathway for "metastatic" is continued for the sake of brevity. The clinician then has to identify whether the patient has metastatic colorectal cancer with "first line", "second line" or "third line and beyond" of therapy (attributes 108, 110 and 112 respectively). In this case, only the pathway for the "second line" is continued. The "second line" is then split into the observations "KRAS/NRAS mutation positive/unknown" or "KRAS/NRAS wild type" in attributes 114 and 116 respectively. Attribute 114 is now split into three different, on-pathway, actions: using "oxaliplatin" 118, using "irinotecan" 120 and using "FOLOFOXIRI" 122.

Example attributes following the "non-metastatic" designation could include "colon-adjuvant" and "rectal". Example attributes for metastatic with "first line" could include "resectable" and "unresectable" .

Each attribute generally leads to a decision node 103 or to a dead end. The dead end may indicate the end of the pathway (e.g. end of treatment) or may indicate a non-expanded attribute (e.g. because it is not relevant to the patient).

Each node 103 shows a decision point for the treatment plan. For example, a patient diagnosed with colorectal cancer may have 'metastasis' with a 'second line' of therapy and with 'KRAS/Mutation Positive'. This patient may then have a choice of three different sets of drugs they could receive, as indicated in the boxes 118, 120 and 122. It could then be up to the clinician to decide which drugs to provide to the patient.

To successfully deploy and implement cancer pathways such as pathway 100, major hospitals and key leadership stakeholders need the ability to analyze the efficacy and performance of these pathways. They need to understand how the physicians are using the pathways as well as when-and-where the physicians digress from the pathways. It is vital to understand its operational bottlenecks and performance issues at/during pathway nodes. Nodes within a pathway are intermediate decision points that govern a particular pathway. Such pathway analytics not only allow key stakeholders to gauge the acceptance, efficacy, and operational usage statistics across all the physicians, but also more importantly, allow them to quantify performance of their pathways and detect and identify reasons for non-adherence.

Exemplary metrics that can quantify the performance and operation of a pathway for an institution may include the total number of patients going through each decision node 103 of the navigation (incl. absolute and/or relative numbers). Nodes with zero to little traffic indicate clinically irrelevant decision steps and may require editing of the pathway or further investigation. Additionally, knowledge of the number of patients going through each pathway can inform the institution of the disease-profile (case mix) of the patients entering their system. This knowledge can provide such institutions with better patient stratification and resource planning.

The number of navigations adhering to the pathway for every disease can also be tracked. This informs the physician/leadership if the plans being prescribed to the patient are on-pathway or off-pathway and allows the leadership to provide an adherence report to the insurance companies for drug pre-authorization, quicker costs re-imbursement and effective negotiation of reimbursement policies. Off-pathway treatment may involve, for exampling, making a decision at a node 103 which is not shown in the clinical pathway 100.

Reasons for going off-pathway can also be tracked to allow institutions to identify where physicians are going off-pathway from the recommended treatment protocols.

Another metric to be tracked may be treatment decisions which are selected most/least often along the course of navigation. This allows institutions to detect and investigate many underlying causes of non-adherence promptly and/or make changes to enhance the treatment protocols.

Obtaining and plotting the above metrics require intensive data mining operations performed by data modelers, extract-transform- load (ETL), designers, and business analysts on team. In addition, these results are displayed in standard graphical charts such as bar charts, pie charts etc. which are non-intuitive, less helpful and time-consuming.

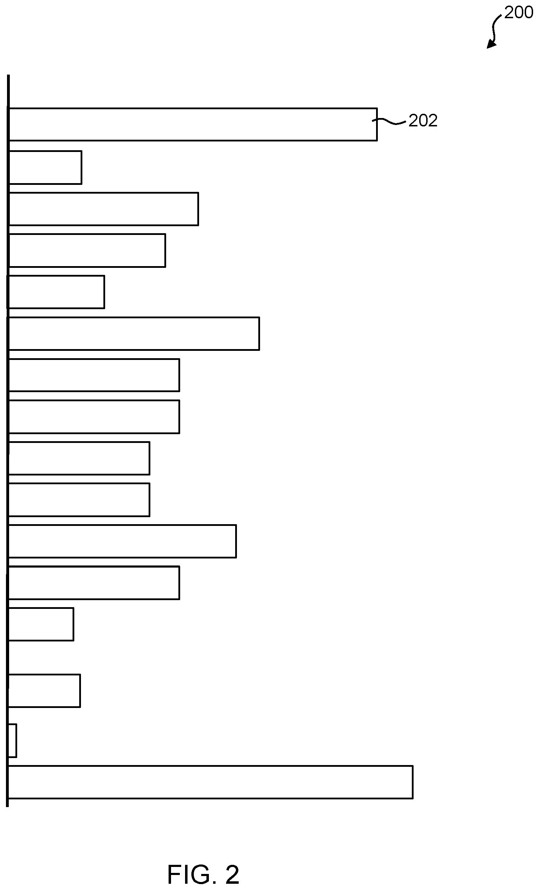

Fig. 2 shows a snapshot of a bar chart 200 showing the number of patients for each treatment-oriented step. Similar to Fig. 1, Fig. 2 is shown to illustrate current methods of showing the analytics for clinical pathways. Each bar 202 is shown to visually track the number of patients in each treatment-oriented step. As previously mentioned, these types of charts are non-intuitive, less helpful and time-consuming.

Moreover, they offer a surface-level snapshot and/or an isolated view into the performance of the pathway-based treatment. Such limitations do not allow for comprehension of the performance of entire pathway at a glance. Rather, these types of charts provide a drilled-down view into a specific navigation branch, hiding the other parts/branches of navigation. The most intuitive representation of a care pathway is considered to be a tree structure as displayed in Fig. 1 since it projects the entire navigation.

Thus, it is proposed to present the aforementioned metrics for every disease's pathway and embed the analytics in an intuitive pathway visualization for quicker and better comprehension for the physicians/clinicians.

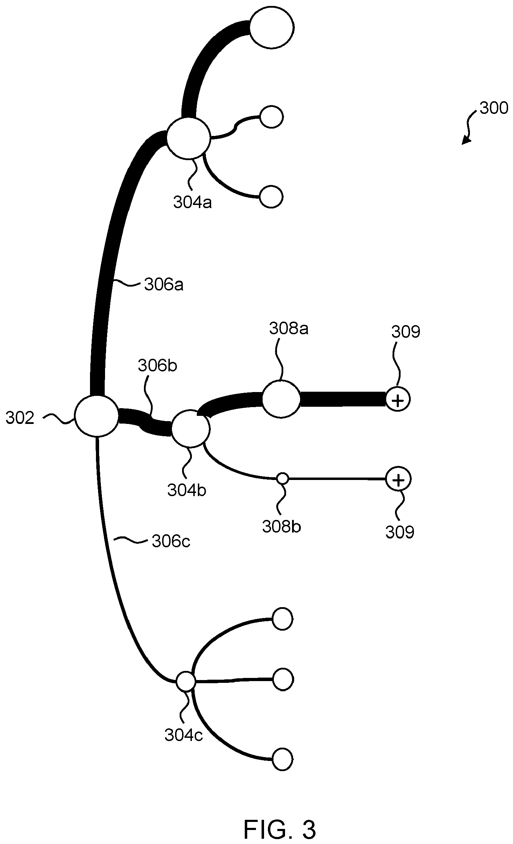

Fig. 3 shows an exemplary clinical pathway 300 according to the invention. Clinical pathway 300 shows the number of patients going between each of the treatment-oriented steps 302, 304 and 308. Each node (treatment-oriented step) represents an attribute, condition, or treatment action within the care pathway which matches the patient's diagnosis.

For example, the number of patients going from treatment-oriented step 302 to treatment-oriented step 304a is shown by the width of the line 306a. Similarly, the number of patients going from treatment-oriented step 302 to treatment-oriented step 304b is shown by the width of the line 306b and the number of patients going from treatment-oriented step 302 to treatment-oriented step 304c is shown by the width of the line 306c.

For treatment-oriented step 304b, the pathway splits into treatment-oriented steps 308a and 308b. Treatment-oriented steps 308a and 308b may be treatment actions for the patient or they may be further attributes of the patient's condition. Additionally, the pathway may be expanded by interacting with the expansion buttons 309 to see further down the pathway.

Thus, just by viewing the clinical pathway 300, the clinician can be shown correlated metrics (e.g. number of patients) of previous patients that have followed particular treatment paths along the clinical pathway.

In this example, the widths of the lines between the nodes (e.g. lines 306a, 306b and 306c) are used to indicate the number of patients going between treatment-oriented steps. Additionally, the color of the nodes and/or the lines may be used to indicate other correlated metrics (e.g. number of patients at the treatment-oriented step being on-pathway or off-pathway). The skilled practitioner would appreciate that the "node" within the context of the present invention has a classical meaning such as the one in graph theory. A graph is made up of vertices (also called nodes or points) which are connected by edges (also called links or lines).

The graphical viewer of the clinical pathway 300 can also augment the original clinical pathway 300 itself, making it highly intuitive. For example, when the clinician clicks on one of the nodes containing the treatment-oriented steps, this may enlarge the clinical pathway 300 to show potential subsequent treatment-oriented steps. In contrast, displaying those correlated metrics using a conventional chart type makes it harder to comprehend where the relevant section in the pathway is. It also presents a very limited view of the pathway and the scope of data.

Furthermore, multiple metrics can be augmented simultaneously (such as number of patients, on/off pathway rate) within the graphical viewer. Furthermore, the graphical viewer is equipped to display these metrics for any pathway, for any decision node and at any level of depth. In contrast, it is extremely complicated to build such a generic analytics framework/platform using standard chart types.

Embedding correlated metrics into the visual representation of the clinical pathway provides a more intuitive visual representation of the clinical pathway 300. This means that the clinician is provided with easily digestible information which will enable to them to make an informed decision on which treatment-oriented step to choose next for the patient.

Some line widths may be linearly related to the number of patients whereas some line widths may be logarithmically related to the number of patients. In general, vertically aligned lines (i.e. lines between a same pair of hierarchical levels along the pathway tree) should all have the same relationship with the corresponding number of patients (i.e. linear, logarithmic etc.).

Exemplary clinical pathways were built in the JavaScript programming language and utilized the D3.js library which is licensed under BSD 3-Clause. In these cases, the models represented oncology pathway data into a specific data structure that allows itself to be rendered as a tree visualization. The data structure has a native specification under the D3.js library specification but has been adapted with several other data types, variables, and attributes to allow the creation and rendition of plots as displayed in Fig. 3. Several functions of JavaScript code have been written to augment the data structure in order to contain calculations which can further be displayed/overlaid on the clinical pathway.

The code makes use of two modules: an analytic module and an interactive visualization module. The data is loaded into the analytic module that is configured by an external configuration file (JSON). This configuration file describes the relation of decision nodes. Based on this described relation, the analytic module builds up the data model in a parent child tree model. The model is not static predefined but can take any shape out of the underlying patient data. This means that pathways can be visualized in any dynamic direction (order).

The configuration file also describes in-exclusion rules to enable filter mechanisms to display cohorts. There are possibilities to augment data into the model; in this case, off-pathway patients with the according reasoning insights.

The visual (D3.js) component is naive and will fully follow the structure of the constructed data model as a rendering engine. A rendering engine also enables interactions such as tooltips and mouse clicks for collapsing, expanding, filtering etc.

Any analytical action required by the visual component is calculated by the analytical component and injected into the visualization module for re-rendering of the state changes through transitions and animations.

A first, relatively important, correlated metric is the total number of patients going through each decision step of the navigation (e.g. absolute or relative numbers) as explained above. This is shown in Fig. 3 by adapting the line widths of the lines between nodes depending on the number of patients going from between the nodes. This correlated metric informs the physicians/clinicians and leadership about the number of treatments delivered at each decision step which allows them to evaluate the treatments which are selected most often and forecast drug utilization. Additionally, this enables the clinicians/leadership to investigate decision points which are never encountered in the pathway navigation and choose treatment navigations which can/cannot be suitable to design clinical trials based on the patient pool/disease-conditions.

Another relevant metric is the adherence to the clinical pathway for every disease. For example, the clinicians/leadership may want to see the reasons why physician go off-pathway from treatment recommendations. Additionally, this enables the physicians to learn which treatments are on-pathway or off-pathway (and why they are off-pathway). Further, the absolute and/or relative numbers of patients going off-pathway may also be shown.

Attributes of the treatment-oriented steps (i.e. pathway metrics) or further correlated metrics between the treatment-oriented steps may be overlaid on the clinical pathway when the clinician hovers over a particular treatment-oriented step, line etc. For example, for an off-pathway treatment-oriented step may overlay a list of the reasons why the off-pathway treatment-oriented step was chosen.

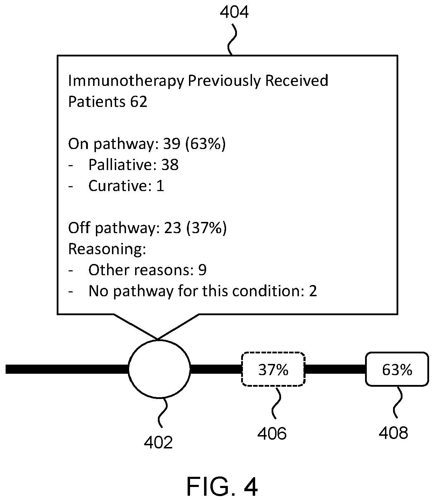

Fig. 4 shows a snapshot of a portion of a clinical pathway showing pathway metrics 404 overlaid on the clinical pathway. The pathway metrics 404 could be shown, for example, when the clinician hovers the mouse over treatment-oriented step 402.

In particular, in this representation, the physicians/clinicians can view that, among the patients which have received immunotherapy, 23 out of 62 (37%) patients receiving a particular treatment were off-pathway. In this case, on-pathway and off-pathway percentages are also shown on the pathway in boxes 408 and 406 respectively.

Several other pathway metrics could also be calculated and overlaid on this graphical visualization of the pathway metrics 404. For example, the performance by costs, performance by survival rates and/or performance by outcomes could also be included in the pathway metrics 404.

Different color coding schemes can also be employed to display different kinds of pathway metrics (and/or correlated metrics) and their gradients. The above representations shown in Figs. 3 and 4 are merely a few examples of analytics embedded on the graphical representation of the clinical pathway.

In general, the idea is to adapt some visual parameters (e.g. line width, color, or gradient, length etc.) of the lines between the nodes to indicate the correlated metrics without the need of additional graphics (e.g. as shown in Fig. 2). The visual parameters applied to the lines (before the user requests more detail) are for preferably non-alphanumeric, i.e. they encode information into a particular visual representation rather than presenting numeric or textual data. Of course, some alphanumeric data may be provided as well, either by default or when requested by a user. The attributes themselves, i.e. the nodes, provide textual information (as shown in Fig. 1) so that the treatment steps and patient conditions along each pathway can be understood. These attributes may for example be textual content provided next to each node. This information is not shown in Fig. 3 to keep the diagram simple and to show only the concept of the invention.

The skilled person would be readily capable of developing a processor for carrying out any herein described method for building a clinical pathway. Thus, each step of a flow chart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

The described disclosure may be provided as a computer program product, or software, that may include a computer-readable storage medium having stored thereon instructions, which may be used to program a computer system (or other electronic devices) to perform a process according to the present disclosure. A computer-readable storage medium includes any mechanism for storing information in a form (e.g., software, processing application) readable by a computer can be used. In various implementations, the processor may be associated with one or more storage media such as volatile and non-volatile computer memory. The computer-readable storage medium may include, but is not limited to, optical storage medium (e.g., CD-ROM), magneto-optical storage medium, read only memory (ROM), random access memory (RAM), erasable programmable memory (e.g., EPROM and EEPROM), flash memory, or other types of medium suitable for storing electronic instructions. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those of ordinary skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. A computer-readable storage medium can be used.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

It is noted that the system and method features described may be used in any combination. In particular, the different features that may be used in any combination are:
the nature of the correlated metrics (e.g. the number of said patients subject to the associated treatment-oriented steps);
the nature of the one or more visual parameters;
the option of determining pathway metrics;
the way pathway metrics are displayed;
the option of determining a recommended pathway.

Any reference signs in the claims should not be construed as limiting the scope.

## Claims

1. A method for generating and displaying clinical pathway analytics (300), the method comprising:
obtaining clinical data corresponding to a pathology in respect of a plurality of patients;
identifying a plurality of treatment-oriented steps (302, 304, 402) from the clinical data forming a plurality of pathways;
determining correlated metrics between treatment-oriented steps in the pathways from the clinical data;
generating the clinical pathway analytics (300) by:
representing the treatment-oriented steps as nodes; and
generating links (306) between the treatment-oriented steps in the pathways, wherein one or more visual parameters of the links correspond to the correlated metrics; and
displaying the clinical pathway analytics (300) to a user.

2. The method of claim 1, wherein the correlated metrics include the number of said patients subject to the associated treatment-oriented steps.

3. The method of claim 1, wherein the one or more visual parameters comprise one or more of:
a line width of the links;
color of the links;
dashed lines; and
doubled lines.

4. The method of claim 1, further comprising:
determining pathway metrics (404) for one or more of the treatment-oriented steps; and
displaying the pathway metrics to the user.

5. The method of claim 4, wherein the pathway metrics comprise one or more of:
whether the treatment-oriented step is on-pathway or off-pathway;
why the treatment-oriented step is off-pathway;
cost of the treatment-oriented step;
survival rate of the treatment-oriented step;
clinical outcomes of the treatment-oriented step;
treatment patterns;
treatment usage data;
population characteristics;
treatment outcomes;
probability score;
outcomes from treatment-oriented step;
cohort identification.

6. The method of claim 4, wherein displaying the pathway metrics comprises overlaying the pathway metrics on top of the corresponding treatment-oriented step (302, 304, 402).

7. The method of claim 1, further comprising:
determining a recommended pathway from the plurality of pathways; and
displaying the treatment-oriented steps or links corresponding to the recommended pathway.

8. A system for generating and displaying clinical pathway analytics (300), the system comprising a processor configured to:
obtain clinical data corresponding to a pathology in respect of a plurality of patients;
identify a plurality of treatment-oriented steps (302, 304, 402) from the clinical data forming a plurality of pathways;
determine correlated metrics between treatment-oriented steps in pathways from the clinical data;
generate the clinical pathway analytics (300) by:
representing the treatment-oriented steps as nodes; and
generating links between the treatment-oriented steps in pathways, wherein one or more visual parameters of the links (306) correspond to the correlated metrics; and
control a display to display the clinical pathway analytics to a user.

9. The system of claim 8, wherein the correlated metrics include the number of said patients subject to the associated treatment-oriented steps.

10. The system of claim 8, wherein the one or more visual parameters comprise one or more of:
a line width of the links;
color of the links;
dashed lines; and
doubled lines.

11. The system of claim 8, wherein the processor is further configured to:
determine pathway metrics (404) for one or more of the treatment-oriented steps; and
displaying the pathway metrics to the user.

12. The system of claim 11, wherein the pathway metrics comprise one or more of:
whether the treatment-oriented step is on-pathway or off-pathway;
why the treatment-oriented step is off-pathway;
cost of the treatment-oriented step;
survival rate of the treatment-oriented step;
clinical outcomes of the treatment-oriented step;
treatment patterns;
treatment usage data;
population characteristics;
treatment outcomes;
probability scores;
outcomes from treatment-oriented step;
cohort identification.

13. The system of claims 11, wherein the processor is configured to display the pathway metrics by overlaying the pathway metrics on top of the corresponding treatment-oriented step (302, 304, 402).

14. The system of claim 8, wherein the processor is further configured to:
determine a recommended pathway from the plurality of pathways; and
display the treatment-oriented steps or links corresponding to the recommended pathway.

15. A computer program comprising computer program code which, when executed on a computing device having a processing system, cause the processing system to perform a method for generating and displaying clinical pathway analytics, the method comprising:
obtaining clinical data corresponding to a pathology in respect of a plurality of patients;
identifying a plurality of treatment-oriented steps (302, 304, 402) from the clinical data forming a plurality of pathways;
determining correlated metrics between treatment-oriented steps in pathways from the clinical data;
generating the clinical pathway analytics (300) by:
representing the treatment-oriented steps as nodes; and
generating links (306) between the treatment-oriented steps in pathways, wherein one or more visual parameters of the links correspond to the correlated metrics; and
displaying the clinical pathway analytics (300) to a user.
